# EUROPEAN PATENT SPECIFICATION

(11) **EP 4 444 412 B1**
(45) Date of publication and mention of the grant of the patent: **14.10.2026**
(21) Application number: 22826421.4
(22) Date of filing: 06.12.2022
(51) Int. Cl.: A61N 1/39, A61B 5/29, A61B 5/00, A61B 5/349

(54) **MODULAR DEFIBRILLATOR SYSTEM**
MODULARES DEFIBRILLATORSYSTEM
SYSTÈME DE DÉFIBRILLATEUR MODULAIRE

(30) Priority: 10.12.2021 US 202163265244 P; 05.12.2022 US 202218061888
(43) Date of publication of application: 16.10.2024
(73) Proprietor: Medtronic, Inc., Minneapolis, Minnesota 55432 (US)
(72) Inventor: ZHANG, Xusheng, Minneapolis, Minnesota 55432 (US); DOLAN, Becky L., Minneapolis, Minnesota 55432 (US); O'BRIEN, Richard J., Minneapolis, Minnesota 55432 (US); LEXCEN, Daniel R., Minneapolis, Minnesota 55432 (US); NIKOLSKI, Vladimir P., Minneapolis, Minnesota 55432 (US); EGGEN, Michael D., Minneapolis, Minnesota 55432 (US)
(74) Representative: Zimmermann & Partner Patentanwälte mbB
(86) International application number: PCT/IB2022/061848
(87) International publication number: WO 2023/105416

(56) References cited:
- CN-A- 112 022 145
- US-A1- 2006 173 498
- US-A1- 2018 243 578
- US-B2- 10 493 290

## Description

### TECHNICAL FIELD

The disclosure relates generally to medical device systems and, more particularly, medical device systems configured to monitor cardiac signals and deliver defibrillation therapy.

### BACKGROUND

Some types of medical devices may be used to monitor one or more physiological parameters of a patient, such as cardiac signals. Such medical devices may include, or may be part of a system that includes, sensors that detect signals associated with such physiological parameters. Values determined based on such signals may be used to assist in detecting changes in patient conditions, in evaluating the efficacy of a therapy, or in general evaluation of patient health. For example, medical devices that monitor physiological parameters may analyze values associated with such physiological parameters in order to identify or monitor a patient condition.

CN 112 022 145 A relates to an external defibrillator device and combined system thereof.

### SUMMARY

The invention is defined by the independent claim 1. This disclosure describes a modular defibrillator system that includes a monitoring device and an external defibrillator device. As will be explained in more detail the below, the system may also include additional devices such as a smartphone for receiving messages and devices to determine if a subject has lost consciousness. An internal cardiac monitoring device (ICMD), may be configured to continuously monitor a patient, e.g., 24 hours per day and 7 days per week, to detect an imminent threat from a coming life-threatening arrhythmia. The monitoring device may alert the subject in advance (e.g., at least 15-30 seconds in advance) so that the subject, or someone else nearby, may retrieve external defibrillator pads, for example from a smartphone case, and put the defibrillator pads on designated locations of the subject's body to ready the subject for treatment of a life-threatening arrhythmia. If the arrhythmia is proven to occur, as confirmed by the monitoring device and/or the external defibrillator device, then the defibrillator may deliver a shock to the patient. In some implementations, the defibrillator may deliver the shock conditionally upon user feedback, or a lack of user feedback, indicating that the user may have lost consciousness. The system (e.g., a smartphone or the defibrillator) may additionally be configured to alert an emergency service, such as 911, that the arrythmia has occurred so that the emergency service can send healthcare professionals to help the subject.

According to one example, a medical system includes an implantable cardiac monitoring device (ICMD) configured to monitor one or more physiological signals of a patient; and in response to detecting, during a first time period, a first pattern in the one or more physiological signals indicative of a current or imminent arrhythmia in the patient, transmit first data to an external user device. The external user device is configured to in response to receiving the first data from the ICMD, outputting a notification of the current or imminent arrythmia; determine, based on second data different than the first data, that a second pattern in the one or more physiological signals during a second time period subsequent to the first period is further indicative of the current or imminent arrhythmia in the patient; and in response to determining that the second pattern in the one or more physiological signals during the second time period subsequent to the first period is further indicative of the current or imminent arrhythmia in the patient, cause an external defibrillator device to deliver a shock to the patient.

According to one example, a device includes transceiver circuitry configured to communicate with an implantable cardiac monitoring device (ICMD); and processing circuitry coupled to the transceiver circuitry and configured to receive from the ICMD first data indicating that the ICMD detected, during a first time period, a first pattern in a first physiological signal that is indicative of a current or imminent arrhythmia in a patient; in response to receiving the first data, output a notification of the current or imminent arrythmia to a user of the device; determine that, during a second time period, a second pattern in a second physiological signal is indicative of the current or imminent arrhythmia in the patient; and in response to determining that the second pattern in the second physiological signal is indicative of the current or imminent arrhythmia in the patient, causing an external defibrillator device to deliver a shock to the patient.

According to one example, a non-claimed method includes receiving, by an external user device from an implantable cardiac monitoring device (ICMD), first data indicating that the ICMD detected, during a first time period, a first pattern in a first physiological signal that is indicative of a current or imminent arrhythmia in a patient; in response to receiving the first data, outputting, by the external user device, a notification of the current or imminent arrythmia to a user of the device; determining by the external user device that, during a second time period, a second pattern in a second physiological signal is indicative of the current or imminent arrhythmia in the patient; and in response to determining that the second pattern in the second physiological signal is indicative of the current or imminent arrhythmia in the patient, causing, by the external user device, an external defibrillator device to deliver a shock to the patient.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF DRAWINGS

FIG. 1A is a conceptual drawing illustrating an example of a modular defibrillator system in conjunction with a patient, in accordance with one or more techniques described herein.
FIG. 1B shows an example timing diagram for operation of the modulator defibrillator system of FIG. 1A.
FIG. 2 is a conceptual drawing illustrating an example configuration of an internal cardiac monitoring device (ICMD) of the medical device system of FIG. 1A, in accordance with one or more techniques described herein.
FIG. 3 is a functional block diagram illustrating an example configuration of the ICMD of FIGS. 1 and 2, in accordance with one or more techniques described herein.
FIGS. 4A and 4B are block diagrams illustrating two additional example ICMDs that may be substantially similar to the ICMD of FIGS. 1-3, but which may include one or more additional features, in accordance with one or more techniques described herein.
FIG. 5 is a block diagram illustrating an example configuration of components of the external user device of FIG. 1A, in accordance with one or more techniques of this disclosure.
FIG. 6 is a block diagram illustrating an example configuration of components of the external defibrillator device of FIG. 1A, in accordance with one or more techniques of this disclosure.
FIG. 7 is a block diagram illustrating an example system that includes an access point, a network, external computing devices, such as a server, and one or more other computing devices, which may be coupled to the ICMD of FIGS. 1-4, a sensing device, and an external user device via a network, in accordance with one or more techniques described herein.
FIG. 8 is a flowchart illustrating an example process that may be performed by the system of FIG. 1.

Like reference characters denote like elements throughout the description and figures.

### DETAILED DESCRIPTION

Sudden cardiac arrest (SCA) due to life-threatening ventricular arrhythmia is one of the leading causes of death in the United States and worldwide. Even with wide deployment of automatic external defibrillators (AEDs), the annual survival rate of people who suffer SCA is still about 5-8% in the United States. An SCA could happen to anybody, anywhere, and at any time without warning. Additionally, many SCAs occur during the night while a subjects is sleeping and/or not in the presence of witnesses or bystanders. For these reasons and others, wide deployment of AEDs alone may still not significantly improve the SCA survival rate.

A high-voltage electrical shock is the most clinically effective way to treat an SCA. Several issues in preventing death or incapacitation due to SCA include (1) knowing if a subject will have or is having a life-threatening arrhythmia, (2) quickly accessing a defibrillator and preparing the defibrillator for high-voltage shock if the subject needs one, and (3) ensuring only necessary shocks are delivered to a subject whose hemodynamics are compromised and who has passed out and thus really needs the therapy. Ensuring high-voltage shock therapy is delivered, in a timely manner, to a subject suffering a life-threatening arrhythmia is one key to increasing the SCA survival rate.

This disclosure describes a modular defibrillator system that includes a monitoring device and an external defibrillator device. As will be explained in more detail the below, the system may also include additional devices such as a smartphone for receiving messages and devices to determine if a subject has lost consciousness. An internal cardiac monitoring device (ICMD), such as the Reveal LINQ^{™} Insertable Cardiac Monitor, available from Medtronic plc, of Dublin, Ireland, may be configured to continuously monitor a patient, e.g., 24 hours per day and 7 days per week, to detect an imminent threat from a coming life-threatening arrhythmia. The monitoring device may alert the subject in advance (e.g., at least 15-30 seconds in advance) so that the subject, or someone else nearby, may retrieve external defibrillator pads, for example from a smartphone case, and put the defibrillator pads on designated locations of the subject's body to ready the subject for treatment of a life-threatening arrhythmia. If the arrhythmia is proven to occur, as confirmed by the monitoring device and/or the external defibrillator device, then the defibrillator may deliver a shock to the patient. In some implementations, the defibrillator may deliver the shock conditionally upon user feedback, or a lack of user feedback, indicating that the user may have lost consciousness. The system (e.g., a smartphone or the defibrillator) may additionally be configured to alert an emergency service, such as 911, that the arrythmia has occurred so that the emergency service can send healthcare professionals to help the subject.

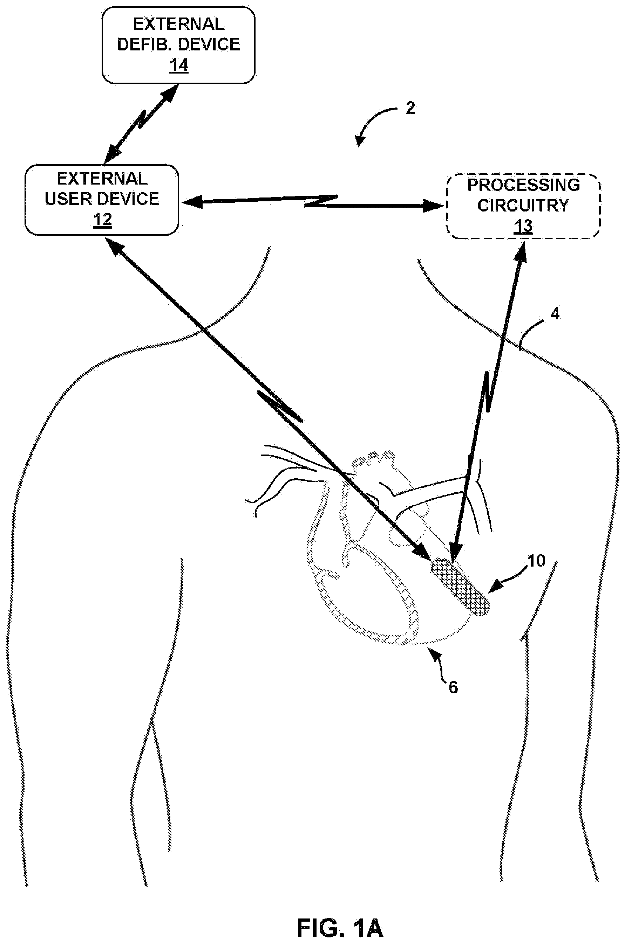

FIG. 1A illustrates the environment of an example modulator defibrillator system 2 in conjunction with a patient 4, in accordance with one or more techniques of this disclosure. The example techniques may be used with an ICMD 10, which may be in communication with an external user device 12 and processing circuitry 13. External user device 12 may additionally be in either wired or wireless communication with external defibrillator device 14.

In the example of FIG. 1A, ICMD 10 is implanted outside of a thoracic cavity of patient 4 (e.g., subcutaneously in the pectoral location illustrated in FIG. 1A) and may be positioned near the sternum near or just below the level of heart 6, e.g., at least partially within the cardiac silhouette. In some examples, ICMD 10 takes the form of a LINQ^{™} ICM. In other examples, ICMD 10 may take a different form and, for example, be configured to deliver therapy, such as pacing therapy. External user device 12 may be a computing device configured for use in settings such as a home, clinic, or hospital, and may further be configured to communicate with ICMD 10 via wireless telemetry. For example, external user device 12 may be coupled to a remote patient monitoring system, such as Carelink^{®}, available from Medtronic plc, of Dublin, Ireland. External user device 12 may, in some examples, include a programmer or an external monitor. Although it is contemplated that external user device 12 may be a proprietary device, it is also contemplated that in many implementations, external user device 12 will be a commercially-available consumer device such as a smartphone, smart watch, or tablet that runs iOS^{®}, Android^{®}, Windows^{®}, or some other such operating system. Processing circuitry 13 may, in some examples, represent processing circuitry located within any combination of ICMD 10 and external user device 12. Additionally, in some examples, processing circuitry 13 may include processing circuitry located within another device or group of devices that are not illustrated in FIG. 1A.

ICMD 10 may include a plurality of electrodes (not illustrated in FIG. 1A) and a set of sensors (not illustrated in FIG. 1A), which collectively detect signals that enable processing circuitry 13 to determine current values of at least one patient parameter associated with patient 4, and evaluate patient 4 for medical conditions (e.g., heart failure, sleep apnea, or chronic obstructive pulmonary disease (COPD)) based on such values. The at least one parameter may include, as examples, any combination of patient motion level, patient posture, subcutaneous tissue impedance, heart rate, heart rate variability, respiration rate, respiration volume, pulse transit time, and temperature. ICMD 10 may additionally be configured to monitor one or more physiological signals, such as a cardiac signal, of patient 4, by, for example, measuring an ECG or EGM of patient 4. In other examples, ICMD 10 may additionally or alternatively be configured to monitor physiological signals such as detecting photoplethysograph (PPG) to detect heart rate, blood pressure, heart sounds or acoustic signals measured by an accelerometer inside ICMD 10, body-temperature, or other such physiological parameters.

According to techniques of this disclosure, ICMD 10 may be configured to monitor one or more physiological signals, such as an ECG, of patient 4. In response to detecting, during a first time period, a first pattern in the one or more physiological signals indicative of a current or imminent arrhythmia in patient 4, ICMD 10 may transmit to external user device 12 first data to cause external user device 12 to output a notification of the current or imminent arrythmia. The first data may, for example, take the form of a computer readable instruction or computer readable message that causes external user device 12 to output the notification.

In response to receiving the first data, external user device 12 may output an audible, visual, or haptic notification to alert the user of external user device 12 that ICMD 10 has detected a pattern in the one or more physiological signals indicative of a current or imminent arrhythmia in patient 4. The notification may, for example, include an audible or visual alert the user or bystander to attach defibrillator pads of external defibrillator device 14 to patient 4. The notification may, for example, also include an indication of locations on patient 4 to attach the defibrillator pads.

After the defibrillator pads of external defibrillator device 14 have been attached to patient 4, in response to processing circuitry 13 detecting a second pattern in the one or more physiological signals during a second time period subsequent to the first period, external user device 12 may cause external defibrillator device 14 to deliver a shock to patient 4.

In some examples, external user device 12 may cause external defibrillator device 14 to deliver a shock to patient 4 dependent on user feedback received at external user device 12. For example, in response to receiving the first data from the ICMD 10, external user device 12 then generate an output indicating that ICMD 10 has detected the arrythmia. The output may, for example, be any sort of audio, visual, or haptic signal to alert a user of the external device that the IMD has detected the arrythmia. External user device 12 may then solicit input from the user. For instance, the user may be able to indicate that the patient is ready to receive a shock or that the patient wishes to delay a shock and wait to see if the arrythmia resolves without a shock. In response to the input from the user, external user device 12 may transmit to external defibrillator device 14, a command to cause external defibrillator device 14 to either deliver the shock to the patient or delay delivery of the shock to the patient. In some implementations, the external device may additionally recommend actions, such as cessation of activity or breathing exercises, for the patient to take to reduce the likelihood of needing a shock. In scenarios where external user device 12 does not receive any sort of user input, then external user device 12 may cause external defibrillator device 14 to deliver the shock, as the lack of such input may indicate that patient 4 has lost consciousness, which is typically indicative of an actual arrythmia.

In some examples, one or both of external user device 12 or ICMD 10 may include accelerometers or other motion detection hardware configured to detect that patient 4 has fallen. External user device 12 may then control external defibrillator device 14 differently in situations where a fall is detected in conjunction with an arrythmia as compared to situations where an arrythmia is detected but no fall is detected. A fall may be an indicator that patient 4 has lost consciousness and is in a more urgent need of a shock. For instance, if a user indicates that patient 4 would like to delay receiving a shock, then external user device 12 may delay the shock, but in response to detecting a fall, however, external user device may cease, or effectively override, the request to delay receiving the shock. In other examples, external user device 12 may cause external defibrillator device 14 to begin charging or to deliver a shock more quickly in a scenario where a fall is detected when compared to a scenario where a fall is not detected.

In some implementations, after external defibrillator device 14 delivers a shock to patient 4, external user device 12 may send to ICMD 10 an indication that the shock has been delivered. In response to receiving such a notification, ICMD 10 may modify a sensing algorithm, by for example, disabling certain morphology-based filters, such as SVT discriminators or noise rejecting filters.

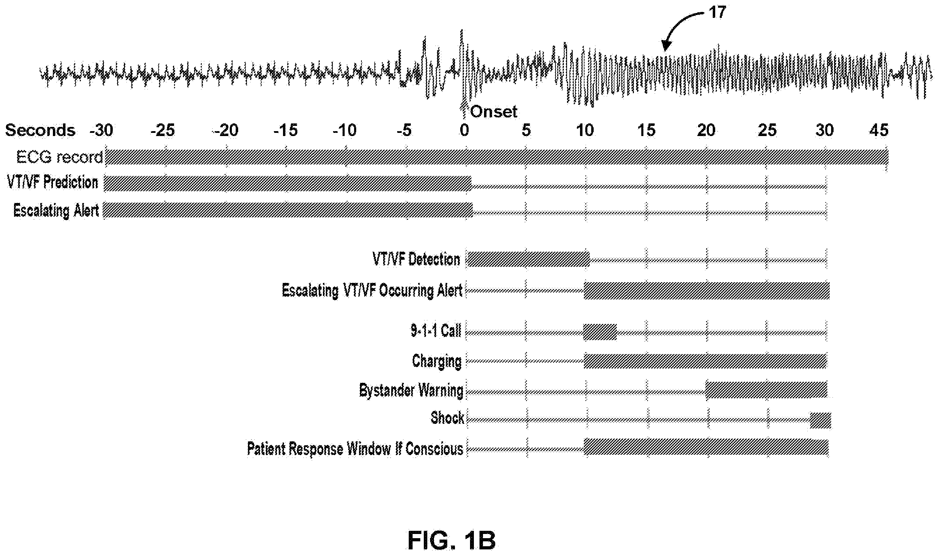

FIG. 1B shows an example timing diagram for modular defibrillator system 2 of FIG. 1A. It should be understood that the timing and durations used in FIG. 1B represent merely one example and that other timing and durations may also be used. In the example of FIG. 1B, ICMD 10 continually records an ECG of patient 4, illustrated in FIG. 1B by ECG signal 17. Patient 4 experiences the onset of VT/VF at time 0 seconds. Approximately 30 seconds prior to patient 4 experiencing the onset of VT/VF, ICMD 10 may be able to perform VT/VF prediction based on the recorded ECG of patient 4 and notify patient 4, via an output of external user device 12, that patient 4 should locate an AED, such as external defibrillator device 14, and attach defibrillator pads to specified locations on the body of patient 4. In some implementations, the intensity of the alert may gradually escalate to signify an increasing likelihood or imminence of a VT/VF event occurring.

Beginning at time 0 seconds, processing circuitry 13 may perform VT/VF detection. In response to the VT/VF detection confirming the existence of a current or imminent arrhythmia in patient 4, external user device 12 may cause external defibrillator device 14 to begin charging in anticipation of external defibrillator device 14 needing to deliver a shock to patient 4. In other examples, external user device 12 may cause external defibrillator device 14 to begin charging earlier, such as during or after VT/VF prediction. In response to the VT/VF detection confirming the existence of a current or imminent arrhythmia in patient 4, external user device 12 may additionally notify emergency medical personnel, such as a healthcare provider or an emergency service, that patient 4 is experiencing an arrythmia. Examples of emergency service include the 9-1-1 emergency service in the United States or the 9-9-9 emergency system in the United Kingdom.

Prior to external defibrillator device 14 delivering a shock to patient 4, external user device 12 and/or external defibrillator device 14 may output warnings to bystanders that patient 4 is about to be shocked. In the example of FIG. 1B, at time t=30 seconds, external defibrillator device 14 delivers a shock to patient 4.

In the example of FIG. 1B, the VT/VF prediction performed by ICMD between -30 seconds and 0 seconds represents an example of ICMD 10 detecting, during a first time period, a first pattern in the one or more physiological signals indicative of a current or imminent arrhythmia in patient 4. The VT/VF prediction performed by processing circuitry 13 between time 0 seconds and 10 seconds represents an example of processing circuitry 13 detecting a second pattern in the one or more physiological signals during a second time period subsequent to the first period. Processing circuitry 13 may continuously monitor the one or more physiological signals, and if at any time, processing circuitry 13 determines that the arrythmia has terminated, processing circuitry 13 may prevent external defibrillator device 14 from delivering a shock to patient 4.

As introduced above, processing circuitry 13 represents processing circuitry that may be located within ICMD 10 or external user device 12 or distributed across ICMD 10 and external user device 12. Processing circuitry 13 may be configured to detect the second pattern in the one or more physiological signals during the second time period subsequent to the first period by, for example, ICMD 10 transmitting raw ECG data to external user device 12, and external user device 12 determining from the raw ECG data that the second pattern in the one or more physiological signals is further indicative of the current or imminent arrhythmia in patient 4. In another example, processing circuitry 13 may be configured to detect the second pattern in the one or more physiological signals during the second time period at ICMD 10, in which case ICMD may transmit to external user device 12 a confirmation that ICMD has detected the second pattern confirming the presence of the current or imminent arrythmia. In yet another example, processing circuitry 13 may be configured to detect the second pattern in the one or more physiological signals during the second time period by external user device 12 receiving raw ECG data from defibrillator pads of external defibrillator device 14, and external user device 12 determining from the raw ECG data that the second pattern is further indicative of the current or imminent arrhythmia in patient 4. Based on detecting the second pattern in the one or more physiological signals during the second time period, processing circuitry 13 may cause external defibrillator device 14 to deliver a shock to patient 4.

In some implementations, external user device 12 may perform a double confirmation prior to causing external defibrillator device 14 to deliver a shock to patient 4. That is, prior to causing external defibrillator device 14 to deliver a shock to patient 4, external user device 12 may determine from raw ECG data received via defibrillator pads of external defibrillator device 14 that the second pattern in the one or more physiological signals is further indicative of the current or imminent arrhythmia in patient 4 and receive confirmation from ICMD 10 that patient 4 is experiencing the current or imminent arrythmia.

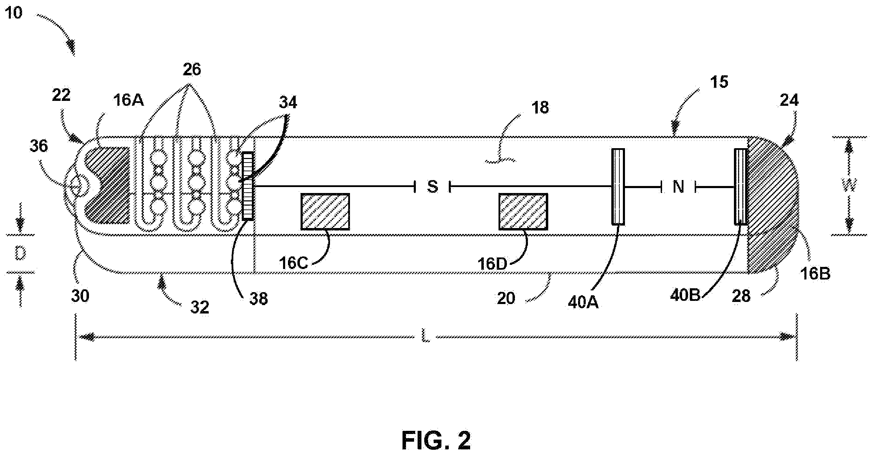

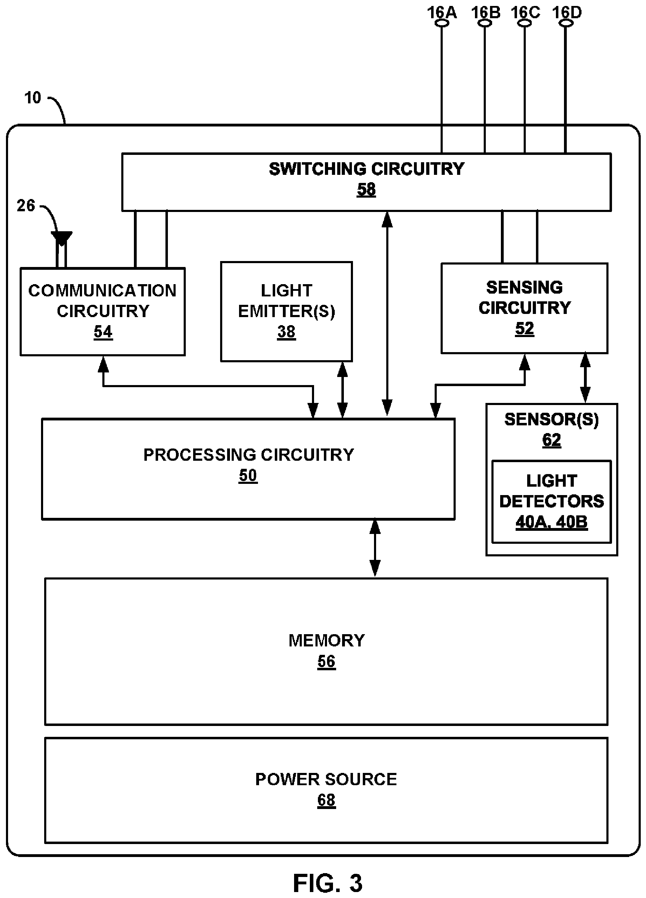

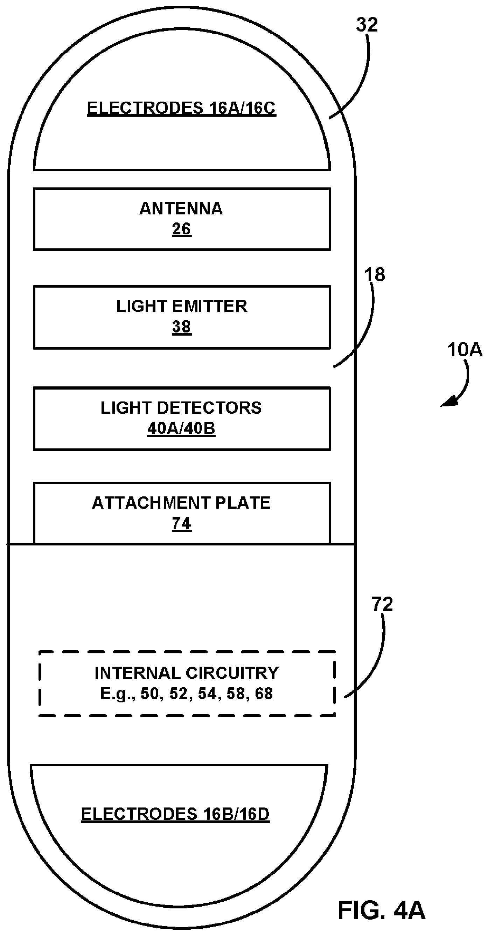

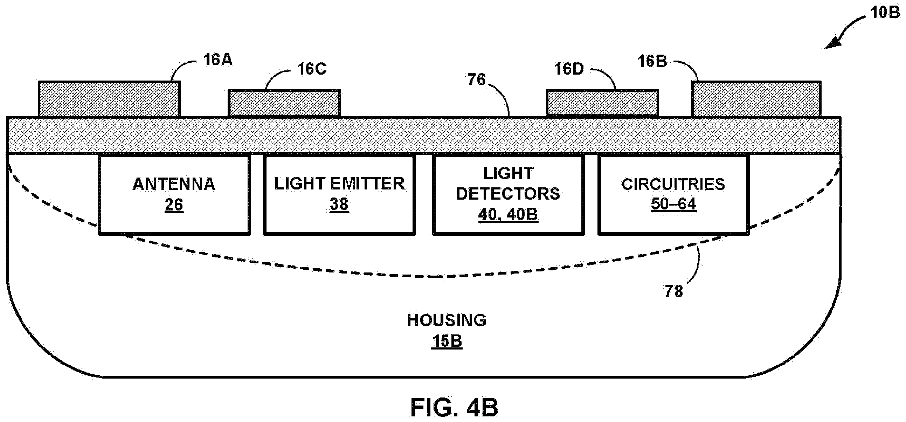

FIGS. 2-4B illustrate various aspects and example arrangements of ICMD 10 of FIG. 1A. For example, FIG. 2 conceptually illustrates an example physical configuration of ICMD 10. FIG. 3 is a block diagram illustrating an example functional configuration of ICMD 10. FIGS. 4A and 4B illustrate additional views of an example physical and functional configuration of ICMD 10. It should be understood that any of the examples of ICMD 10 described below with respect to FIGS. 2-4B may be used to implement the techniques described herein for determining whether to perform parameter measurements using ICMD 10. ICMD 10 may concurrently collect sensor signals and parameter measurements. For example, compiled data may be used for model fitting and other means of Artificial Intelligence to assess and predict a trajectory of a patient condition. Additionally, compiled data may be used to categorize or group parameter measurements of ICMD 10. Some measurements made by of ICMD 10 may be used to categorize or group other measurements.

FIG. 2 is a conceptual drawing illustrating an example configuration of ICMD 10 of the medical device system 2 of FIG. 1A, in accordance with one or more techniques described herein. In the example shown in FIG. 2, ICMD 10 may include a leadless, subcutaneously-implantable monitoring device having housing 15, proximal electrode 16A, and distal electrode 16B. housing 15 may further include first major surface 18, second major surface 20, proximal end 22, and distal end 24. In some examples, ICMD 10 may include one or more additional electrodes 16C, 16D positioned on one or both of major surfaces 18, 20 of ICMD 10. Housing 15 encloses electronic circuitry located inside the ICMD 10, and protects the circuitry contained therein from fluids such as body fluids. In some examples, electrical feedthroughs provide electrical connection of electrodes 16A-16D, and antenna 26, to circuitry within housing 15. In some examples, electrode 16B may be formed from an uninsulated portion of conductive housing 15.

In the example shown in FIG. 2, ICMD 10 is defined by a length L, a width W, and thickness or depth D. In this example, ICMD 10 is in the form of an elongated rectangular prism in which length L is significantly greater than width W, and in which width W is greater than depth D. However, other configurations of ICMD 10 are contemplated, such as those in which the relative proportions of length L, width W, and depth D vary from those described and shown in FIG. 2. In some examples, the geometry of the ICMD 10, such as the width W being greater than the depth D, may be selected to allow ICMD 10 to be inserted under the skin of patient 4 using a minimally invasive procedure and to remain in the desired orientation during insertion. In addition, ICMD 10 may include radial asymmetries (e.g., the rectangular shape) along a longitudinal axis of ICMD 10, which may help maintain the device in a desired orientation following implantation.

In some examples, a spacing between proximal electrode 16A and distal electrode 16B may range from about 30-55 millimeters (mm), about 35-55 mm, or about 40-55 mm, or more generally from about 25-60 mm. Overall, ICMD 10 may have a length L of about 20-30 mm, about 40-60 mm, or about 45-60 mm. In some examples, the width W of major surface 18 may range from about 3-10 mm, and may be any single width or range of widths between about 3-10 mm. In some examples, a depth D of ICMD 10 may range from about 2-9 mm. In other examples, the depth D of ICMD 10 may range from about 2-5 mm, and may be any single or range of depths from about 2-9 mm. In any such examples, ICMD 10 is sufficiently compact to be implanted within the subcutaneous space of patient 4 in the region of a pectoral muscle. In some examples, a housing of ICMD 10 is configured for ECG measurements. Additionally, in some examples, a housing of ICMD 10 is configured for optical sensing. A housing configured for optical sensing may be significantly smaller than a housing configured for ECG sensing, and the housing configured for optical sensing may be implanted in different regions throughout the body of patient 4 than the housing configured for ECG sensing.

ICMD 10, according to an example of the present disclosure, may have a geometry and size designed for ease of implant and patient comfort. Examples of ICMD 10 described in this disclosure may have a volume of 3 cubic centimeters (cm³) or less, 1.5 cm³ or less, or any volume therebetween. In addition, in the example shown in FIG. 2, proximal end 22 and distal end 24 are rounded to reduce discomfort and irritation to surrounding tissue once implanted under the skin of patient 4. In some examples, power may be supplied to ICMD 10 through inductive coupling.

In some examples, first major surface 18 of ICMD 10 faces outward towards the skin, when ICMD 10 is inserted within patient 4, whereas second major surface 20 is faces inward toward musculature of patient 4. Thus, first and second major surfaces 18, 20 may face in directions along a sagittal axis of patient 4 (see FIG. 1A), and this orientation may be maintained upon implantation due to the dimensions of ICMD 10. In some examples, first major surface 18 faces inward towards the musculature of patient 4, and second major surface 20 faces outward towards the skin of patient 4.

Proximal electrode 16A and distal electrode 16B may be used to sense cardiac EGM signals (e.g., ECG signals) when ICMD 10 is implanted subcutaneously in patient 4. Processing circuitry 13 may determine a pulse transit time value based in part on cardiac ECG signals, as further described below. In some examples, processing circuitry of ICMD 10 also may determine whether cardiac ECG signals of patient 4 are indicative of arrhythmia or other abnormalities, which processing circuitry of ICMD 10 may evaluate in determining whether a condition (e.g., heart failure) of patient 4 has changed. The cardiac ECG signals may be stored in a memory of the ICMD 10, and data derived from the cardiac ECG signals may be transmitted via integrated antenna 26 to another medical device, such as external user device 12. In some examples, one or both of electrodes 16A and 16B also may be used to detect a subcutaneous impedance value for assessing a congestion status of patient 4, monitoring one or more respiratory parameters (e.g., respiration rate, respiration rate variability respiration effort, mechanical functions, and relative tidal volume), and/or may be used by communication circuitry of ICMD 10 for tissue conductance communication (TCC) with external user device 12. In some examples, ECG and/or impedance signals obtained by electrodes 16A and 16B may be used to determine one or more respiratory parameters (e.g., respiration rate, respiration rate variability, respiration effort, mechanical functions, or relative tidal volume).

In the example shown in FIG. 2, proximal electrode 16A is in close proximity to proximal end 22, and distal electrode 16B is in close proximity to distal end 24 of ICMD 10. In this example, distal electrode 16B is not limited to a flattened, outward facing surface, but may extend from first major surface 18, around rounded edges 28 or end surface 30, and onto the second major surface 20 in a three-dimensional curved configuration. As illustrated, proximal electrode 16A is located on first major surface 18 and is substantially flat and outward facing. However, in other examples not shown here, proximal electrode 16A and distal electrode 16B both may be configured like proximal electrode 16A shown in FIG. 2, or both may be configured like distal electrode 16B shown in FIG. 2. In some examples, additional electrodes 16C and 16D may be positioned on one or both of first major surface 18 and second major surface 20, such that a total of four electrodes are included on ICMD 10. Any of electrodes 16A-16D may be formed of a biocompatible conductive material. For example, any of electrodes 16A-16D may be formed from any of stainless steel, titanium, platinum, iridium, or alloys thereof. In addition, electrodes of ICMD 10 may be coated with a material such as titanium nitride or fractal titanium nitride, although other suitable materials and coatings for such electrodes may be used.

In the example shown in FIG. 2, proximal end 22 of ICMD 10 includes header assembly 32 having one or more of proximal electrode 16A, integrated antenna 26, anti-migration projections 34, and suture hole 36. Integrated antenna 26 is located on the same major surface (e.g., first major surface 18) as proximal electrode 16A, and may be an integral part of header assembly 32. In other examples, integrated antenna 26 may be formed on the major surface opposite from proximal electrode 16A, or, in still other examples, may be incorporated within housing 15 of ICMD 10. Antenna 26 may be configured to transmit or receive electromagnetic signals for communication. For example, antenna 26 may be configured to transmit to or receive signals from a programmer via inductive coupling, electromagnetic coupling, tissue conductance, NFC, Radio Frequency Identification (RFID), Bluetooth ^{®}, WiFi, LoRa, or other proprietary or non-proprietary wireless telemetry communication schemes. Antenna 26 may be coupled to communication circuitry of ICMD 10, which may drive antenna 26 to transmit signals to external user device 12 and may transmit signals received from external user device 12 to processing circuitry of ICMD 10 via communication circuitry.

ICMD 10 may include several features for retaining ICMD 10 in position once subcutaneously implanted in patient 4. For example, as shown in FIG. 2, housing 15 may include anti-migration projections 34 positioned adjacent integrated antenna 26. Anti-migration projections 34 may include a plurality of bumps or protrusions extending away from first major surface 18, and may help prevent longitudinal movement of ICMD 10 after implantation in patient 4. In other examples, anti-migration projections 34 may be located on the opposite major surface as proximal electrode 16A and/or integrated antenna 26. In addition, in the example shown in FIG. 2 header assembly 32 includes suture hole 36, which provides another means of securing ICMD 10 to patient 4 to prevent movement following insertion. In the example shown, suture hole 36 is located adjacent to proximal electrode 16A. In some examples, header assembly 32 may include a molded header assembly made from a polymeric or plastic material, which may be integrated or separable from the main portion of ICMD 10.

In some examples, processing circuitry of ICMD 10 may determine a subcutaneous tissue impedance value of patient 4 based on signals received from at least two of electrodes 16A-16D. For example, processing circuitry of ICMD 10 may generate one of a current or voltage signal, deliver the signal via a selected two or more of electrodes 16A-16D, and measure the resulting other of current or voltage. Processing circuitry of ICMD 10 may determine an impedance signal based on the delivered current or voltage and the measured voltage or current.

In the example shown in FIG. 2, ICMD 10 includes light emitter(s) 38 and a proximal light detector 40A and a distal light detector 40B (collectively, "light detectors 40") positioned on housing 15 of ICMD 10. Light detector 40A may be positioned at a distance S from light emitter(s) 38, and a distal light detector 40B positioned at a distance S+N from light emitter(s) 38. In other examples, ICMD 10 may include only one of light detectors 40A, 40B, or may include additional light emitters and/or additional light detectors. Collectively, light emitter(s) 38 and light detectors 40A, 40B may include an optical sensor, which may, for example, be used to determine StO₂ or SpO₂ values of patient 4.

In some examples, ICMD 10 may include one or more additional sensors, such as one or more accelerometers or gyroscopes (not shown). Such accelerometers may be 3D accelerometers configured to generate signals indicative of one or more types of movement of the patient, such as gross body movement (e.g., activity) of the patient, patient posture, movements associated with the beating of the heart, or coughing, rales, or other respiration abnormalities. Additionally, or alternatively, one or more of the parameters monitored by ICMD 10 may fluctuate in response to changes in one or more such types of movement. For example, changes in parameter values sometimes may be attributable to increased patient activity (e.g., exercise or other physical activity as compared to inactivity) or to changes in patient posture, and not necessarily to changes in a heart failure status caused by a progression of a heart failure condition. Thus, in some methods of identifying or monitoring a condition of patient 4, it may be advantageous to account for such fluctuations when determining whether a change in a patient parameter is indicative of a change in a condition of patient 4.

FIG. 3 is a functional block diagram illustrating an example configuration of ICMD 10 of FIGS. 1 and 2, in accordance with one or more techniques described herein. In the illustrated example, ICMD 10 includes electrodes 16, antenna 26, light emitter(s) 38, processing circuitry 50, sensing circuitry 52, communication circuitry 54, memory 56, switching circuitry 58, sensors 62 including light detectors 40, and power source 68. In some examples, memory 56 includes computer-readable instructions that, when executed by processing circuitry 50, cause ICMD 10 and processing circuitry 50 to perform various functions attributed to ICMD 10 and processing circuitry 50 herein. Memory 56 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media.

Processing circuitry 50 may include fixed function circuitry and/or programmable processing circuitry. Processing circuitry 50 may include any one or more of a microprocessor, a controller, a digital signal processor (DSP), an application specific integrated circuit (ASIC), a field-programmable gate array (FPGA), or equivalent discrete or analog logic circuitry. In some examples, processing circuitry 50 may include multiple components, such as any combination of one or more microprocessors, one or more controllers, one or more DSPs, one or more ASICs, or one or more FPGAs, as well as other discrete or integrated logic circuitry. The functions attributed to processing circuitry 50 herein may be embodied as software, firmware, hardware or any combination thereof.

Sensing circuitry 52 and communication circuitry 54 may be selectively coupled to electrodes 16A-16D via switching circuitry 58, as controlled by processing circuitry 50. Sensing circuitry 52 may monitor signals from electrodes 16A-16D in order to monitor electrical activity of heart (e.g., to produce an ECG), and/or subcutaneous tissue impedance. Sensing circuitry 52 also may monitor signals from sensors 62, which may include light detectors 40A, 40B, and any additional light detectors that may be positioned on ICMD 10. In some examples, sensing circuitry 52 may include one or more filters and amplifiers for filtering and amplifying signals received from one or more of electrodes 16A-16D and/or light detectors 40A, 40B. Although not explicitly shown in FIG. 3, sensors 62 may additionally include impedance sensors, accelerometers, a gyroscope, a PPG sensor, a tissue oxygen sensor, a blood pressure monitor, respiration rate sensors, respiration effort sensors, respiration pattern sensor, a temperature sensor, or other such sensors.

Communication circuitry 54 may include any suitable hardware, firmware, software or any combination thereof for communicating with another device, such as external user device 12 or another ICMD or sensor, such as a pressure sensing device. Under the control of processing circuitry 50, communication circuitry 54 may receive downlink telemetry from, as well as send uplink telemetry to, external user device 12 or another device with the aid of an internal or external antenna, e.g., antenna 26. In some examples, communication circuitry 54 may communicate with external user device 12. In addition, processing circuitry 50 may communicate with a networked computing device via an external device (e.g., external user device 12) and a computer network, such as the Medtronic CareLink^{®} Network developed by Medtronic, plc, of Dublin, Ireland.

A clinician or other user may retrieve data from ICMD 10 using external user device 12, or by using another local or networked computing device configured to communicate with processing circuitry 50 via communication circuitry 54. The clinician may also program parameters of ICMD 10 using external user device 12 or another local or networked computing device.

Power source 68 is configured to deliver operating power to the components of ICMD 10. Power source 68 may include one or more batteries and a power generation circuit to produce the operating power. In some examples, the one or more batteries may include a battery that is rechargeable to allow extended operation. In some examples, recharging is accomplished through proximal inductive interaction between an external charger and an inductive charging coil within external user device 12. Power source 68 may include any one or more of a plurality of different battery types, such as nickel cadmium batteries and lithium ion batteries. A non-rechargeable battery may be selected to last for several years, while a rechargeable battery may be inductively charged from an external device, e.g., on a daily or weekly basis. In some examples, power source 68 may include both a rechargeable battery and a non-rechargeable battery.

FIGS. 4A and 4B are block diagrams illustrating two additional example ICMDs that may be substantially similar to ICMD 10 of FIGS. 1-3, but which may include one or more additional features, in accordance with one or more techniques described herein. The components of FIGS. 4A and 4B may not necessarily be drawn to scale, but instead may be enlarged to show detail. FIG. 4A is a block diagram of a top view of an example configuration of an ICMD 10A. FIG. 4B is a block diagram of a side view of example ICMD 10B, which may include an insulative layer as described below.

FIG. 4A is a conceptual drawing illustrating another example ICMD 10A that may be substantially similar to ICMD 10 of FIG. 1A. In addition to the components illustrated in FIGS. 1-3, the example of ICMD 10 illustrated in FIG. 4A also may include a body portion 72 and an attachment plate 74. Attachment plate 74 may be configured to mechanically couple header assembly 32 to body portion 72 of ICMD 10A. Body portion 72 of ICMD 10A may be configured to house one or more of the internal components of ICMD 10 illustrated in FIG. 3, such as one or more of processing circuitry 50, sensing circuitry 52, communication circuitry 54, memory 56, switching circuitry 58, and internal components of sensors 62. In some examples, body portion 72 may be formed of one or more of titanium, ceramic, or any other suitable biocompatible materials.

FIG. 4B is a conceptual drawing illustrating another example ICMD 10B that may include components substantially similar to ICMD 10 of FIG. 1A. In addition to the components illustrated in FIGS. 1-3, the example of ICMD 10B illustrated in FIG. 4B also may include a wafer-scale insulative cover 76, which may help insulate electrical signals passing between electrodes 16A-16D and/or optical detectors 40A, 40B on housing 15B and processing circuitry 50. In some examples, insulative cover 76 may be positioned over an open housing 15 to form the housing for the components of ICMD 10B. One or more components of ICMD 10B (e.g., antenna 26, light emitter(s) 38, light detectors 40A, 40B, processing circuitry 50, sensing circuitry 52, communication circuitry 54, switching circuitry 58, or any combination thereof ) may be formed on a bottom side of insulative cover 76, such as by using flip-chip technology. Insulative cover 76 may be flipped onto a housing 15B. When flipped and placed onto housing 15B, the components of ICMD 10B formed on the bottom side of insulative cover 76 may be positioned in a gap 78 defined by housing 15B.

Insulative cover 76 may be configured so as not to interfere with the operation of ICMD 10B. For example, one or more of electrodes 16A-16D may be formed or placed above or on top of insulative cover 76, and electrically connected to switching circuitry 58 through one or more vias (not shown) formed through insulative cover 76.

In some examples, light emitter(s) 38 may include an optical filter between light emitter(s) 38 and insulative cover 76, which may limit the spectrum of emitted light to be within a narrow band. Similarly, light detectors 40A, 40B may include optical filters between light detectors 40A, 40B and insulative cover 76, so that light detectors 40A, 40B detects light from a narrow spectrum, generally at longer wavelengths than the emitted spectrum. Other optical elements that may be included in the ICMD 10B may include index matching layers, antireflective coatings, or optical barriers, which may be configured to block light emitted sideways by the light emitter(s) 38 from reaching light detectors 40.

Although not explicitly shown in FIGS. 2-4B, in some examples, ICMD 10 may be further configured to deliver pacing therapy to patient 4. In one particular example, ICMD 10 may be configured to deliver pacing therapy, such as anti-tachycardia pacing therapy, to patient 4 in response to, or only in response to, receiving from external user device 12 a confirmation that external defibrillator device 14 is attached to patient 4 and ready to deliver a shock if needed. Furthermore, in some examples, ICMD 10 may include more or fewer sensors or sensing capabilities than illustrated in the examples of FIGS. 2-4B. For example, in some implementations, ICMD 10 may not include optical sensors and/or perform impedance sensing.

It should be understood that the examples provided above represent one example implementation of ICMD 10. In other implementations, ICMD 10 may have a different shape or configuration and may, for example, include more than two electrodes and support more than one sensing vector.

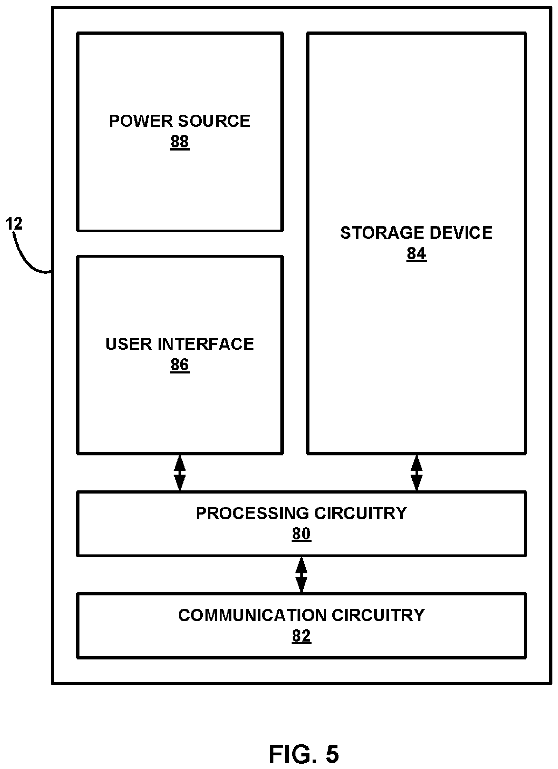

FIG. 5 is a block diagram illustrating an example configuration of components of external user device 12, in accordance with one or more techniques of this disclosure. In the example of FIG. 5, external user device 12 includes processing circuitry 80, communication circuitry 82, storage device 84, user interface 86, and power source 88.

Processing circuitry 80, in one example, may include one or more processors that are configured to implement functionality and/or process instructions for execution within external user device 12. For example, processing circuitry 80 may be capable of processing instructions stored in storage device 84. Processing circuitry 80 may include, for example, microprocessors, DSPs, ASICs, FPGAs, or equivalent discrete or integrated logic circuitry, or a combination of any of the foregoing devices or circuitry. Accordingly, processing circuitry 80 may include any suitable structure, whether in hardware, software, firmware, or any combination thereof, to perform the functions ascribed herein to processing circuitry 80.

Communication circuitry 82, also referred to herein as transceiver circuitry, may include any suitable hardware, firmware, software or any combination thereof for communicating with other devices, such as ICMD 10 and external defibrillator device 14. Under the control of processing circuitry 80, communication circuitry 82 may receive downlink telemetry from, as well as send uplink telemetry to, ICMD 10, or another device via, for example, Bluetooth or Bluetooth Low Energy. Under the control of processing circuitry 80, communication circuitry 82 may also send data to and receive data from external defibrillator device 14. Communication circuitry 82 may, for example, wirelessly communicate with external defibrillator device 14 over Bluetooth, WiFi, or any other such wireless protocol. Communication circuitry 82 may, additionally or alternatively, include a port, such as a micro-USB port, USB-C port, lighting port, or other such port, to facilitate wired communication between external user device 12 and external defibrillator device 14. Communication circuitry 82 may also be able to communicate via one or more cellular communication standards, such as 4G, 4G-LTE (Long-Term Evolution), LTE Advanced, 5G, or the like.

Storage device 84 may be configured to store information within external user device 12 during operation. Storage device 84 may include a computer-readable storage medium or computer-readable storage device. In some examples, storage device 84 includes one or more of a short-term memory or a long-term memory. Storage device 84 may include, for example, RAM, DRAM, SRAM, magnetic discs, optical discs, flash memories, or forms of EPROM or EEPROM. In some examples, storage device 84 is used to store data indicative of instructions for execution by processing circuitry 80. Storage device 84 may be used by software or applications running on external user device 12 to temporarily store information during program execution.

A user, such as a clinician or patient 4, may interact with external user device 12 through user interface 86. User interface 86 includes a display (not shown), such as an LCD or LED display or other type of screen, with which processing circuitry 80 may present information related to ICMD 10 (e.g., EGM signals obtained from at least one electrode or at least one electrode combination). In addition, user interface 86 may include an input mechanism to receive input from the user. The input mechanisms may include, for example, any one or more of buttons, a keypad (e.g., an alphanumeric keypad), a peripheral pointing device, a touch screen, or another input mechanism that allows the user to navigate through user interfaces presented by processing circuitry 80 of external user device 12 and provide input. In other examples, user interface 86 also includes audio circuitry for providing audible notifications, instructions or other sounds to patient 4, receiving voice commands from patient 4, or both. Storage device 84 may include instructions for operating user interface 86 and for managing power source 88.

Power source 88 is configured to deliver operating power to the components of external user device 12. Power source 88 may include a battery and a power generation circuit to produce the operating power. In some examples, the battery is rechargeable to allow extended operation. Recharging may be accomplished by electrically coupling power source 88 to a cradle or plug that is connected to an alternating current (AC) outlet. In addition, recharging may be accomplished through proximal inductive interaction between an external charger and an inductive charging coil within external user device 12. In other examples, traditional batteries (e.g., nickel cadmium or lithium ion batteries) may be used. In addition, external user device 12 may be directly coupled to an alternating current outlet to operate.

Data exchanged between external user device 12 and ICMD 10 may include ECG data. External user device 12 may transmit data including computer readable instructions which, when implemented by ICMD 10, may control ICMD 10 to change one or more operational parameters and/or export collected data. For example, processing circuitry 80 may transmit an instruction to ICMD 10 which requests ICMD 10 to export collected data to external user device 12. In turn, external user device 12 may receive the collected data from ICMD 10 and store the collected data in storage device 84. Additionally, or alternatively, processing circuitry 80 may export instructions to ICMD 10 requesting ICMD 10 to update electrode combinations for stimulation or sensing. External user device 12 may also receive instructions or messages from ICMD 10, including alerts from ICMD 10 that ICMD 10 has detected a pattern in one or more physiological signals indicative of a current or imminent arrhythmia in a patient.

External user device 12 may also be configured to receive data from and transmit data to external defibrillator device 14. For example, after external user device 12 receives a message from ICMD 10 indicating that ICMD 10 has detected a pattern in one or more physiological signals indicative of a current or imminent arrhythmia in a patient and after a patient has attached pads of external defibrillator device 14, external user device 12 may receive, from external defibrillator device 14, ECG data.

According to techniques of this disclosure, processing circuitry 80 may receive, from ICMD 10 via communications circuitry 82, first data indicating that ICMD 10 detected, during a first time period, a first pattern in a first physiological signal that is indicative of a current or imminent arrhythmia in a patient. In response to receiving the first data indicating that ICMD 10 has either detected a current arrythmia or precited an imminent arrythmia, processing circuitry 80 may output, via user interface 86, a notification of the current or imminent arrythmia to a user of the device. The notification may, for example, be any sort of audible, visual, or haptic notification to alert the user of external user device 12 that ICMD 10 has predicted a current or imminent arrhythmia is occurring or about to occur. The notification may, for example, include an audible alert via a speaker of external user device 12 or a visual alert via user interface 86 that instructs a user of the device as to how and where to attach defibrillator pads of external defibrillator device 14 to a patient.

Upon confirming that the arrythmia initially detected or predicted by ICMD 10 has progressed into an ongoing arrythmia in patient 4, processing circuitry 80 may be configured to cause external defibrillator device 14 to deliver a shock to patient 4. In some examples, processing circuitry 13 may receive confirmation based on an analysis performed by ICMD 10 or external defibrillator device 14. For example, processing circuitry 13 may receive, from the ICMD 10, second data indicating that ICMD 10 detected, during a second time period, a second pattern in the second physiological signal that confirms that patient 4 is experiencing an ongoing arrythmia.

In other examples, processing circuitry 80 may confirm that the arrythmia initially detected or predicted by ICMD 10 has progressed into an ongoing arrythmia based on analysis of a cardiac signal of patient 4 received from ICMD 10 or from external defibrillator device 14. Processing circuitry 80 may, for example, determine that a second physiological signal received from ICMD 10 or external defibrillator device 14 includes a second pattern indicative of the current or imminent arrhythmia in patient 4.

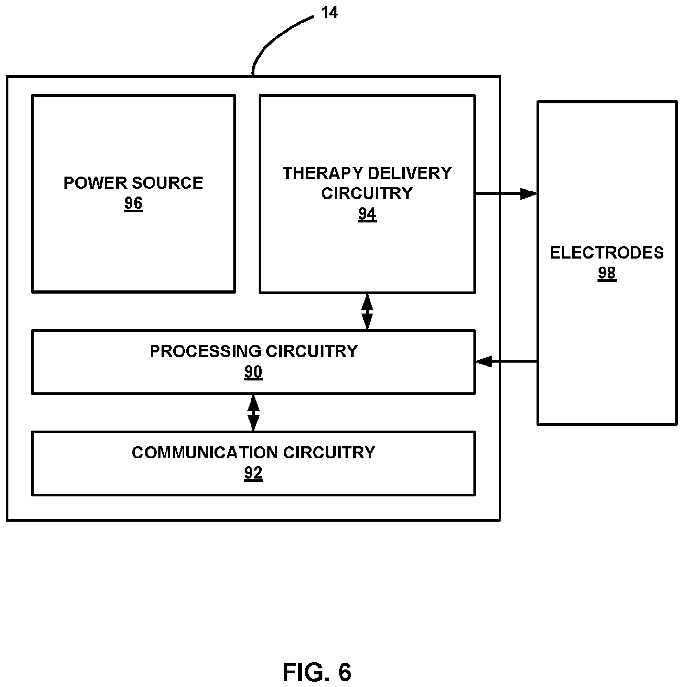

FIG. 6 is a block diagram illustrating an example configuration of components of external defibrillator device 14, in accordance with one or more techniques of this disclosure. In the example of FIG. 6, external defibrillator device 14 includes processing circuitry 90, communication circuitry 92, therapy delivery circuitry 94, and power source 96.

Processing circuitry 90, in one example, may include one or more processors that are configured to implement functionality and/or process instructions for execution within external defibrillator device 14. For example, processing circuitry 90 may be capable of processing instructions and/or may include, for example, microprocessors, DSPs, ASICs, FPGAs, or equivalent discrete or integrated logic circuitry, or a combination of any of the foregoing devices or circuitry. Accordingly, processing circuitry 90 may include any suitable structure, whether in hardware, software, firmware, or any combination thereof, to perform the functions ascribed herein to processing circuitry 90.

In some implementations it is contemplated that processing circuitry 90 may be relatively sophisticated circuitry capable of analyzing ECG data to detect and identify arrythmias. In other examples, however, it is contemplated that processing circuitry 90 may be relatively simple circuitry, and that advanced functions such as analyzing ECG data to detect and identify arrythmias or making therapy delivery decisions may be performed primarily by processing circuitry 50 of ICMD 10 or processing circuitry 80 of external user device 12.

Communication circuitry 92, also referred to herein as transceiver circuitry, may include any suitable hardware, firmware, software or any combination thereof for communicating with other devices, such external user device 12. Under the control of processing circuitry 90, communication circuitry 92 may, for example, receive instructions from external user device 12 or transmit data, such as ECG data, to external user device 12. Communication circuitry 92 may, for example, be configured to facilitate wireless communication, via Bluetooth or WiFi, with external user device 12 or be configured to facilitate wired communication with a port, such as a micro-USB port, USB-C port, lighting port, or other such port, of external user device 12.

Therapy delivery circuitry 94 is configured to deliver, via electrodes 98, therapy to a patient. Therapy delivery circuitry 94 may, for instance, include a variety of capacitors, transformers, switches, and the like configured to deliver a defibrillation or cardioversion shock to the heart of patient 4 via electrodes 98, which represent two or more electrodes placed on external surfaces of the body of patient 4.

Power source 98 is configured to deliver operating power to the components of external defibrillator device 14. Power source 98 may include a battery and a power generation circuit to produce the operating power. In some examples, the battery is rechargeable to allow extended operation. Recharging may be accomplished by electrically coupling power source 98 to a cradle or plug that is connected to an alternating current (AC) outlet. In addition, recharging may be accomplished through proximal inductive interaction between an external charger and an inductive charging coil within external user device 12. In other examples, traditional batteries (e.g., nickel cadmium or lithium ion batteries) may be used. In addition, external user device 12 may be directly coupled to an alternating current outlet to operate. In some examples, power source 96 may be configured to receive power from power source 86 of external user device 12.

In one example implementation, external defibrillator device 14 may take the form of a phone case that surrounds a phone. The phone may, for example, be external user device 12. A back cover of the phone case may enclose electrodes that can be connected to patient 4. Other components of external defibrillator device 14, such as processing circuitry 90, communication circuitry 92, therapy delivery circuitry 94, and power source 96 may be distributed throughout the phone case.

In other examples, external defibrillator device 14 may be a relatively small standalone device that can be worn by patient 4 on a belt or carried by patient 4 in a backpack or pocket. In yet other examples, external defibrillator device 14 may be a more conventional, portable AED.

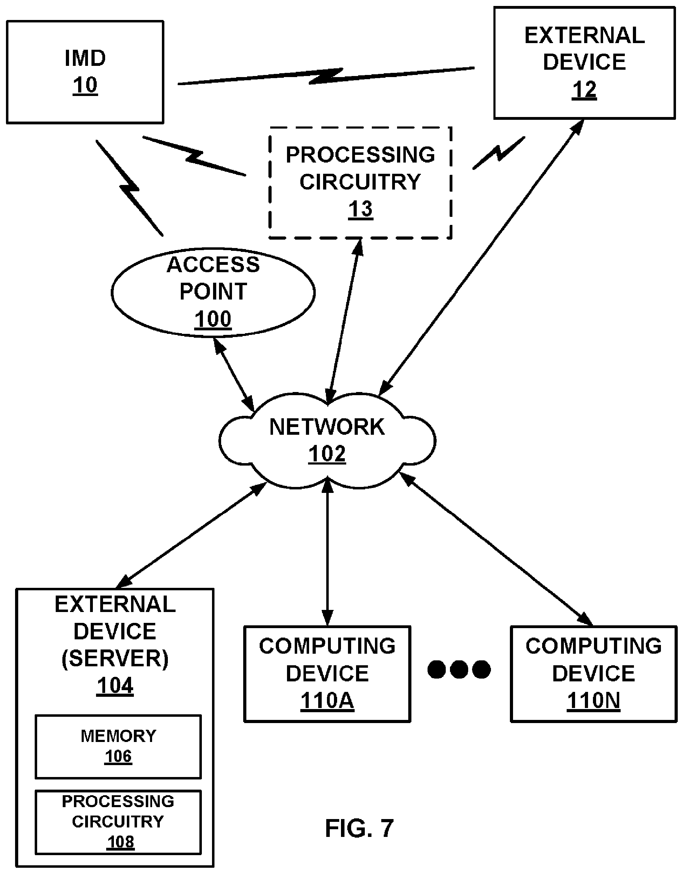

FIG. 7 is a block diagram illustrating an example system that includes an access point 100, a network 102, external computing devices, such as a server 104, and one or more other computing devices 110A-110N, which may be coupled to ICMD 10, external user device 12, and processing circuitry 13 via network 102, in accordance with one or more techniques described herein. In this example, ICMD 10 may use communication circuitry 54 to communicate with external user device 12 via a first wireless connection, and to communication with an access point 100 via a second wireless connection. In the example of FIG. 7, access point 100, external user device 12, server 104, and computing devices 110A-110N are interconnected and may communicate with each other through network 102.

Access point 100 may include a device that connects to network 102 via any of a variety of connections, such as telephone dial-up, digital subscriber line (DSL), or cable modem connections. In other examples, access point 100 may be coupled to network 102 through different forms of connections, including wired or wireless connections. In some examples, access point 100 may be a user device, such as a tablet or smartphone, that may be co-located with the patient. As discussed above, ICMD 10 may be configured to transmit data, such as current values and heart failure statuses, to external user device 12. In addition, access point 100 may interrogate ICMD 10, such as periodically or in response to a command from the patient or network 102, in order to retrieve current values or heart failure statuses determined by processing circuitry 50 of ICMD 10, or other operational or patient data from ICMD 10. Access point 100 may then communicate the retrieved data to server 104 via network 102.

In some cases, server 104 may be configured to provide a secure storage site for data that has been collected from ICMD 10, and/or external user device 12. In some cases, server 104 may assemble data in web pages or other documents for viewing by trained professionals, such as clinicians, via computing devices 110A-110N. One or more aspects of the illustrated system of FIG. 7 may be implemented with general network technology and functionality, which may be similar to that provided by the Medtronic CareLink^{®} Network developed by Medtronic plc, of Dublin, Ireland.

In some examples, one or more of computing devices 110A-110N (e.g., device 110A) may be a tablet or other smart device located with a clinician, by which the clinician may program, receive alerts from, and/or interrogate ICMD 10. For example, the clinician may access parameter values associated with patient 4 through device 110A, such as when patient 4 is in in between clinician visits, to check on a heart failure status of patient 4 as desired. In some examples, the clinician may enter instructions for a medical intervention for patient 4 into an app in device 110A, such as based on a heart failure status of patient 4 determined by ICMD 10, or based on other patient data known to the clinician. Device 110A then may transmit the instructions for medical intervention to another of computing devices 110A-110N (e.g., device 110B) located with patient 4 or a caregiver of patient 4. For example, such instructions for medical intervention may include an instruction to change a drug dosage, timing, or selection, to schedule a visit with the clinician, or to seek medical attention. In further examples, device 110B may generate an alert to patient 4 based on a heart failure status of patient 4 determined by processing circuitry 13, which may enable patient 4 proactively to seek medical attention prior to receiving instructions for a medical intervention. In this manner, patient 4 may be empowered to take action, as needed, to address his or her heart failure status, which may help improve clinical outcomes for patient 4.

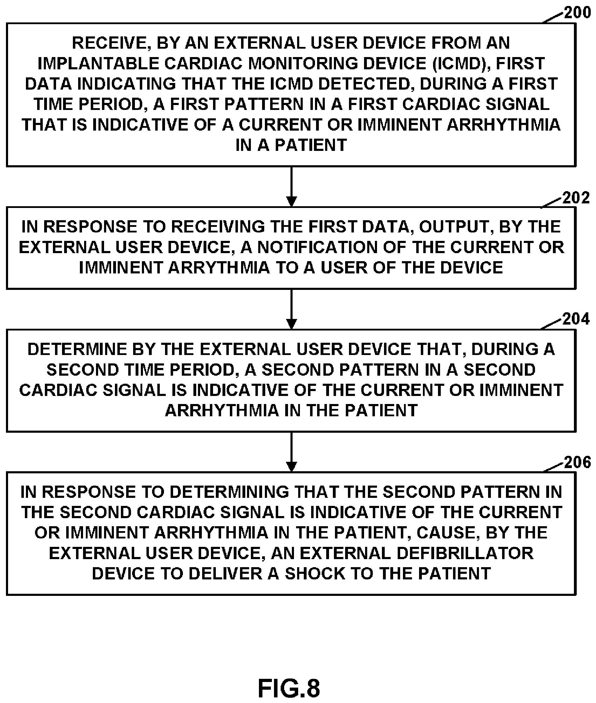

FIG. 8 is a flowchart illustrating an example process that may be performed by the system of FIG. 1. In the example of FIG. 8, external user device 12 receives from ICMD 10 first data indicating that ICMD 10 detected, during a first time period, a first pattern in a first one or more physiological, e.g., cardiac, signals that is indicative of a current or imminent arrhythmia in patient 4 (200). A first physiological signal may, for example, be an ECG signal monitored by ICMD 10.

In response to receiving the first data, ICMD 10 outputs a notification of the current or imminent arrythmia to a user of external user device 12 (202). The notification may, for example, be an alert to attach defibrillator pads to patient 4 and may include an indication of locations, e.g., body locations, on patient 4 to attach the defibrillator pads.

External user device 12 determines that, during a second time period, a second pattern in a second physiological signal is indicative of the current or imminent arrhythmia in patient 4 (204). In one example, to determine that the second pattern in the second physiological signal is indicative of the current or imminent arrhythmia in patient 4, external user device may receive, from ICMD 10, the second data indicating that ICMD 10 detected, during the second time period, the second pattern in the second physiological signal that is indicative of the current or imminent arrhythmia in patient 4. In another example, external user device 12 may also receive, via defibrillator pads, such as electrodes 98 of external defibrillator device 14, a third cardiac signal and determine that, during a third time period, a third pattern in the third cardiac signal is indicative of the current or imminent arrhythmia in patient 4. In response to determining that the second pattern in the second physiological signal is indicative of the current or imminent arrhythmia in patient 4 and the third pattern in the third cardiac signal is indicative of the current or imminent arrhythmia in patient 4, external user device 12 causes external defibrillator device 14 to deliver the shock to patient 4. The third time period may fully or partially overlap with the second time period.

In another example, to determine that the second pattern in the second physiological signal is indicative of the current or imminent arrhythmia in patient 4, external user device 12 may receive, via electrodes 98 of external defibrillator 14, the second physiological signal and detect the second pattern in the second physiological signal. That is, external user device, e.g., processing circuitry 80, may receive raw ECG data and execute one or more algorithms to recognize the second pattern and determine that the second pattern is indicative of the current or imminent arrhythmia in patient 4.

In response to determining that the second pattern in the second physiological signal is indicative of the current or imminent arrhythmia in patient 4, external user device 12 may cause external user device 12 to begin a charging process during which, for example, power source 96 or power source 86 charges therapy delivery circuitry 94 in anticipation of therapy delivery circuitry 94 delivering a shock to patient 4. In some examples, the charging process may be initiated in response to external user device 12 receiving the first data from ICMD 10 detected a current or imminent arrhythmia in patient 4. The charging process may, for example, be aborted if ICMD 10 or external user device 12 determines that the arrythmia has terminated or if a user of external user device 12 indicates that patient 4 is not experiencing symptoms or is otherwise maintaining consciousness.

In response to determining that the second pattern in the second physiological signal is indicative of the current or imminent arrhythmia in patient 4, external user device 12 causes external defibrillator device 14 to deliver a shock to patient 4 (206). External user device 12 may, for example, send a command, via either a wired or wireless connection, to external defibrillator device 14 to cause the external defibrillator device 14 to deliver the shock to patient 4. In some examples, determining that the second pattern in the second physiological signal is indicative of the current or imminent arrhythmia in patient 4 may represent an ongoing process that is repeated by external user device 12 many times prior to external user device 12 causing external defibrillator device 14 to deliver a shock to patient 4.

The techniques described in this disclosure may be implemented, at least in part, in hardware, software, firmware, or any combination thereof. For example, various aspects of the techniques may be implemented within one or more microprocessors, DSPs, ASICs, FPGAs, or any other equivalent integrated or discrete logic QRS circuitry, as well as any combinations of such components, embodied in external devices, such as physician or patient programmers, stimulators, or other devices. The terms "processor" and "processing circuitry" may generally refer to any of the foregoing logic circuitry, alone or in combination with other logic circuitry, or any other equivalent circuitry, and alone or in combination with other digital or analog circuitry.

For aspects implemented in software, at least some of the functionality ascribed to the systems and devices described in this disclosure may be embodied as instructions on a computer-readable storage medium such as RAM, DRAM, SRAM, magnetic discs, optical discs, flash memories, or forms of EPROM or EEPROM. The instructions may be executed to support one or more aspects of the functionality described in this disclosure.

In addition, in some aspects, the functionality described herein may be provided within dedicated hardware and/or software modules. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components. Also, the techniques could be fully implemented in one or more circuits or logic elements. The techniques of this disclosure may be implemented in a wide variety of devices or apparatuses, including an ICMD, an external programmer, a combination of an ICMD and external programmer, an integrated circuit (IC) or a set of ICs, and/or discrete electrical circuitry, residing in an ICMD and/or external programmer.

## Claims

1. A medical system comprising:
an implantable cardiac monitoring device, ICMD, (10) configured to:
monitor one or more physiological signals of a patient; and
in response to detecting, during a first time period, a first pattern in the one or more physiological signals indicative of a current or imminent arrhythmia in the patient, transmit first data to an external user device;
the external user device (12, 14), wherein the external user device is configured to:
in response to receiving the first data from the ICMD, outputting (202) a notification of the current or imminent arrhythmia;
determine (204), based on second data different than the first data, that a second pattern in the one or more physiological signals during a second time period subsequent to the first period is further indicative of the current or imminent arrhythmia in the patient; and
in response to determining that the second pattern in the one or more physiological signals during the second time period subsequent to the first period is further indicative of the current or imminent arrhythmia in the patient, cause (206) an external defibrillator device to deliver a shock to the patient.

2. The system of claim 1, wherein the external user device comprises is either the external defibrillator device (14) or is in wired communication with the external defibrillator device (14).

3. The system of any one of claims 1 or 2, wherein the external user device comprises a first battery and the external defibrillator device comprises a second battery that is separate from the first battery.

4. The system of any one of claims 1 or 2, wherein the external user device comprises a battery and the battery is configured to provide power to the external defibrillator device.

5. The system of any one of claims 1-4, wherein the ICMD is configured to communicate with the external defibrillator device via the external user device.

6. The system of any one of claims 1-5, wherein the external user device comprises one of a smartphone device or a smartwatch device.

7. The system of any one of claims 1-6, wherein the second data comprises ECG data and to determine that the second pattern in the one or more physiological signals during the second time period subsequent to the first period is further indicative of the current or imminent arrhythmia in the patient, the external user device is further configured to:
receive the ECG data from the ICMD (10); and
determine from the ECG data that the second pattern in the one or more physiological signals is further indicative of the current or imminent arrhythmia in the patient.

8. The system of any one of claims 1-7, wherein:
the ICMD (10) is configured to determine that the second pattern in the one or more physiological signals is further indicative of the current or imminent arrhythmia in the patient; and
in response to determining that the second pattern in the one or more physiological signals is further indicative of the current or imminent arrhythmia in the patient, transmit the second data to the external user device to cause the external user device to cause the external defibrillator device to deliver a shock to the patient.

9. The system of any one of claims 1-8, wherein the external user device (12,14) is further configured to:
receive, via defibrillator pads of the external defibrillator device, a third cardiac signal;
determine that, during a third time period, a third pattern in the third cardiac signal is indicative of the current or imminent arrhythmia in the patient; and
in response to receiving the second data and determining that the third pattern in the third cardiac signal is indicative of the current or imminent arrhythmia in the patient, causing the external defibrillator device to deliver a shock to the patient.

10. The system of claim 9, wherein the third time period fully or partially overlaps with the second time period.

11. The system of any one of claims 1-10, wherein the notification comprises one or more of an alert to attach defibrillator pads to the patient, an indication of locations on the patient to attach the defibrillator pads, oran alert to a care provider to the existence of the current or imminent arrhythmia in the patient.

12. The system of any one of claims 1-11, wherein the one or more physiological signals comprises one or more ECG signals.

13. The system of any one of claims 1-12, wherein the ICMD (10) is further configured to:
receive, from the external user device, a confirmation that the external defibrillator device is ready to deliver the shock to the patient; and
only in response to receiving the confirmation, deliver pacing therapy to the patient.

14. The system of any one of claims 1-13, wherein the external user device (12,14) is configured to transmit to the ICMD (10) an indication of how many shocks have been delivered to the patient by the external defibrillator device.

15. The system of any one of claims 1-14, wherein to determine that the second pattern in the second physiological signal is indicative of the current or imminent arrhythmia in the patient, the external device is further configured to:
receive, via defibrillator pads of the external defibrillator device, the second physiological signal; and
detect the second pattern in the second physiological signal.

## Patentansprüche

1. Medizinisches System, umfassend:
eine implantierbare Herzüberwachungsvorrichtung, ICMD, (10), die ausgelegt ist zum:
Überwachen eines oder mehrerer physiologischer Signale eines Patienten; und
Übertragen von ersten Daten an eine externe Benutzervorrichtung in Reaktion auf Detektieren eines ersten Musters in dem einen oder den mehreren physiologischen Signalen während eines ersten Zeitraums, das eine aktuelle oder bevorstehende Arrhythmie in dem Patienten angibt;
die externe Benutzervorrichtung (12, 14), wobei die externe Benutzervorrichtung ausgelegt ist zum:
Ausgeben (202) einer Benachrichtigung über die aktuelle oder bevorstehende Arrhythmie in Reaktion auf Empfangen der ersten Daten von der ICMD;
Bestimmen (204), dass ein zweites Muster in dem einen oder den mehreren physiologischen Signalen während eines zweiten Zeitraums, der auf den ersten Zeitraum folgt, weiter die aktuelle oder bevorstehende Arrhythmie in dem Patienten angibt, basierend auf zweiten Daten, die sich von den ersten Daten unterscheiden; und
Veranlassen (206) einer externen Defibrillatorvorrichtung, einen Schock an den Patienten abzugeben, in Reaktion auf Bestimmen, dass das zweite Muster in dem einen oder den mehreren physiologischen Signalen während des zweiten Zeitraums, der auf den ersten Zeitraum folgt, weiter die aktuelle oder bevorstehende Arrhythmie in dem Patienten angibt.

2. System nach Anspruch 1, wobei die externe Benutzervorrichtung entweder die externe Defibrillatorvorrichtung (14) umfasst oder ist, oder in drahtgebundener Kommunikation mit der externen Defibrillatorvorrichtung (14) steht.

3. System nach einem der Ansprüche 1 oder 2, wobei die externe Benutzervorrichtung eine erste Batterie umfasst und die externe Defibrillatorvorrichtung eine zweite Batterie umfasst, die separat von der ersten Batterie vorliegt.

4. System nach einem der Ansprüche 1 oder 2, wobei die externe Benutzervorrichtung eine Batterie umfasst und die Batterie dazu ausgelegt ist, der externen Defibrillatorvorrichtung Leistung bereitzustellen.

5. System nach einem der Ansprüche 1-4, wobei die ICMD dazu ausgelegt ist, über die externe Benutzervorrichtung mit der externen Defibrillatorvorrichtung zu kommunizieren.

6. System nach einem der Ansprüche 1-5, wobei die externe Benutzervorrichtung eines von einer Smartphone-Vorrichtung oder einer Smartwatch-Vorrichtung umfasst.

7. System nach einem der Ansprüche 1-6, wobei die zweiten Daten EKG-Daten umfassen und die externe Benutzervorrichtung zum Bestimmen, dass das zweite Muster in dem einen oder den mehreren physiologischen Signalen während des zweiten Zeitraums, der auf den ersten Zeitraum folgt, weiter die aktuelle oder bevorstehende Arrhythmie in dem Patienten angibt, ferner ausgelegt ist zum:
Empfangen der EKG-Daten von der ICMD (10); und
Bestimmen aus den EKG-Daten, dass das zweite Muster in dem einen oder den mehreren physiologischen Signalen weiter die aktuelle oder bevorstehende Arrhythmie in dem Patienten angibt.

8. System nach einem der Ansprüche 1-7, wobei:
die ICMD (10) dazu ausgelegt ist, zu bestimmen, dass das zweite Muster in dem einen oder den mehreren physiologischen Signalen weiter die aktuelle oder bevorstehende Arrhythmie in dem Patienten angibt; und
in Reaktion auf Bestimmen, dass das zweite Muster in dem einen oder den mehreren physiologischen Signalen weiter die aktuelle oder bevorstehende Arrhythmie in dem Patienten angibt, die zweiten Daten an die externe Benutzervorrichtung überträgt, um zu veranlassen, dass die externe Benutzervorrichtung veranlasst, dass die externe Defibrillatorvorrichtung einen Schock an den Patienten abgibt.

9. System nach einem der Ansprüche 1-8, wobei die externe Benutzervorrichtung (12, 14) ferner ausgelegt ist zum:
Empfangen eines dritten Herzsignals über Defibrillatorpads der externen Defibrillatorvorrichtung; Bestimmen, dass während eines dritten Zeitraums ein drittes Muster in dem dritten Herzsignal die aktuelle oder bevorstehende Arrhythmie in dem Patienten angibt; und
Veranlassen, dass die externe Defibrillatorvorrichtung einen Schock an den Patienten abgibt, in Reaktion auf Empfangen der zweiten Daten und Bestimmen, dass das dritte Muster in dem dritten Herzsignal die aktuelle oder bevorstehende Arrhythmie in dem Patienten angibt.

10. System nach Anspruch 9, wobei der dritte Zeitraum vollständig oder teilweise mit dem zweiten Zeitraum überlappt.

11. System nach einem der Ansprüche 1-10, wobei die Benachrichtigung eine oder mehrere von einer Warnung zum Anbringen von Defibrillatorpads an dem Patienten, einer Angabe von Orten zum Anbringen der Defibrillatorpads an dem Patienten oder einer Warnung an einen Versorgungsanbieter umfasst, dass die aktuelle oder bevorstehende Arrhythmie in dem Patienten vorliegt.

12. System nach einem der Ansprüche 1-11, wobei das eine oder die mehreren physiologischen Signale ein oder mehrere EKG-Signale umfassen.

13. System nach einem der Ansprüche 1-12, wobei die ICMD (10) ferner ausgelegt ist zum:
Empfangen einer Bestätigung von der externen Benutzervorrichtung, dass die externe Defibrillatorvorrichtung bereit ist, den Schock an den Patienten abzugeben; und
Abgeben von Stimulationstherapie an den Patienten nur in Reaktion auf Empfangen der Bestätigung.

14. System nach einem der Ansprüche 1-13, wobei die externe Benutzervorrichtung (12, 14) dazu ausgelegt ist, an die ICMD (10) eine Angabe zu übertragen, wie viele Schocks durch die externe Defibrillatorvorrichtung an den Patienten abgegeben wurden.

15. System nach einem der Ansprüche 1-14, wobei zum Bestimmen, dass das zweite Muster in dem zweiten physiologischen Signal die aktuelle oder bevorstehende Arrhythmie in dem Patienten angibt, die externe Vorrichtung ferner ausgelegt ist zum:
Empfangen des zweiten physiologischen Signals über Defibrillatorpads der externen Defibrillatorvorrichtung; und
Detektieren des zweiten Musters in dem zweiten physiologischen Signal.

## Revendications

1. Système médical comprenant :
un dispositif de suivi cardiaque implantable, ICMD, (10) configuré pour :
surveiller un ou plusieurs signaux physiologiques d'un patient ; et
en réponse à la détection, pendant une première période de temps, d'un premier motif dans le ou les signaux physiologiques indiquant une arythmie courante ou imminente chez le patient, transmettre des premières données à un dispositif utilisateur externe ;
le dispositif utilisateur externe (12, 14), le dispositif utilisateur externe étant configuré pour :
en réponse à la réception des premières données de l'ICMD, émettre en sortie (202) une notification de l'arythmie actuelle ou imminente ;
déterminer (204), sur la base de secondes données différentes des premières données, qu'un deuxième motif dans le ou les signaux physiologiques pendant une deuxième période de temps suivant la première période indique en outre l'arythmie actuelle ou imminente chez le patient ; et
en réponse à la détermination que le deuxième motif dans le ou les signaux physiologiques pendant la deuxième période de temps suivant la première période indique en outre l'arythmie actuelle ou imminente chez le patient, un dispositif défibrillateur externe (206) administre un choc au patient.

2. Système selon la revendication 1, le dispositif utilisateur externe soit comprenant le dispositif défibrillateur externe (14), soit étant en communication filaire avec le dispositif défibrillateur externe (14).

3. Système selon l'une quelconque des revendications 1 ou 2, le dispositif utilisateur externe comprenant une première batterie et le dispositif défibrillateur externe comprenant une seconde batterie qui est séparée de la première batterie.

4. Système selon l'une quelconque des revendications 1 ou 2, le dispositif utilisateur externe comprenant une batterie et la batterie étant configurée pour fournir de l'énergie au dispositif défibrillateur externe.

5. Système selon l'une quelconque des revendications 1 à 4, l'ICMD étant configuré pour communiquer avec le dispositif défibrillateur externe via le dispositif utilisateur externe.

6. Système selon l'une quelconque des revendications 1 à 5, le dispositif utilisateur externe comprenant l'un d'un dispositif de smartphone ou d'un dispositif de montre intelligente.

7. Système selon l'une quelconque des revendications 1 à 6, les secondes données comprenant des données ECG et pour déterminer que le deuxième motif dans le ou les signaux physiologiques pendant la deuxième période de temps suivant la première période est en outre indicatif de l'arythmie actuelle ou imminente chez le patient, le dispositif utilisateur externe étant en outre configuré pour :
recevoir les données ECG de l'ICMD (10) ; et
déterminer à partir des données ECG que le deuxième motif dans le ou les signaux physiologiques indique en outre l'arythmie actuelle ou imminente chez le patient.

8. Système selon l'une quelconque des revendications 1 à 7,
l'ICMD (10) étant configuré pour déterminer que le deuxième motif dans le ou les signaux physiologiques est en outre indicatif de l'arythmie actuelle ou imminente chez le patient ; et
en réponse à la détermination que le deuxième motif dans le ou les signaux physiologiques indique en outre l'arythmie actuelle ou imminente chez le patient, transmettre les secondes données au dispositif utilisateur externe pour amener le dispositif utilisateur externe à provoquer l'administration d'un choc par le dispositif défibrillateur externe au patient.

9. Système selon l'une quelconque des revendications 1 à 8, le dispositif utilisateur externe (12,14) étant en outre configuré pour :
recevoir, via les électrodes de défibrillation du dispositif défibrillateur externe, un troisième signal cardiaque ;
déterminer que, pendant une troisième période de temps, un troisième motif dans le troisième signal cardiaque indique l'arythmie actuelle ou imminente chez le patient ; et
en réponse à la réception des secondes données et à la détermination que le troisième motif dans le troisième signal cardiaque indique l'arythmie actuelle ou imminente chez le patient, le dispositif défibrillateur externe administre un choc au patient.

10. Système selon la revendication 9, la troisième période de temps chevauchant totalement ou partiellement la deuxième période de temps.

11. Système selon l'une quelconque des revendications 1 à 10, la notification comprenant un ou plusieurs éléments parmi une alerte pour attacher des électrodes de défibrillation au patient, une indication des emplacements sur le patient pour attacher les électrodes de défibrillation, ou une alerte pour un fournisseur de soins de l'existence de l'arythmie actuelle ou imminente chez le patient.

12. Système selon l'une quelconque des revendications 1 à 11, le ou les signaux physiologiques comprenant un ou plusieurs signaux ECG.

13. Système selon l'une quelconque des revendications 1 à 12, l'ICMD (10) étant en outre configuré pour :
recevoir, du dispositif utilisateur externe, une confirmation que le dispositif défibrillateur externe est prêt à administrer le choc au patient ; et
uniquement en réponse à la confirmation, administrer la thérapie de stimulation au patient.

14. Système selon l'une quelconque des revendications 1 à 13, le dispositif utilisateur externe (12, 14) étant configuré pour transmettre à l'ICMD (10) une indication du nombre de chocs qui ont été administrés au patient par le dispositif défibrillateur externe.

15. Système selon l'une quelconque des revendications 1 à 14, pour déterminer que le deuxième motif dans le deuxième signal physiologique est indicatif de l'arythmie actuelle ou imminente chez le patient, le dispositif externe étant en outre configuré pour :
recevoir, par l'intermédiaire des électrodes de défibrillation du dispositif défibrillateur externe, le deuxième signal physiologique ; et
détecter le deuxième motif dans le deuxième signal physiologique.
